# EUROPÄISCHE PATENTSCHRIFT

(11) **EP 3 610 822 B1**
(45) Veröffentlichungstag und Bekanntmachung des Hinweises auf die Patenterteilung: **07.07.2021**
(21) Anmeldenummer: 18000672.8
(22) Anmeldetag: 14.08.2018
(51) Int. Cl.: A61B 90/00, A61B 17/00

(54) **MARKIERUNGSKÖRPER UND VERFAHREN ZUR HERSTELLUNG VON DIESEM MARKIERUNGSKÖRPER**
MARKING BODY AND METHOD FOR PRODUCTION OF THIS MARKING BODY
CORPS DE MARQUAGE ET PROCÉDÉ DE FABRICATION DE CE CORPS DE MARQUAGE

(43) Veröffentlichungstag der Anmeldung: 19.02.2020
(73) Patentinhaber: Endosmart Gesellschaft für Medizintechnik mbH, 76297 Stutensee (DE)
(72) Erfinder: VOGEL, Bernd, 76199 Karlsruhe (DE)
(74) Vertreter: Mehl-Mikus, Claudia

(56) Entgegenhaltungen:
- WO-A1-2017/207777
- DE-T2- 60 116 956
- US-A1- 2007 135 826
- US-A1- 2014 046 347
- US-A1- 2014 371 778

## Beschreibung

Die Erfindung betrifft einen Markierungskörper, ausgebildet zur Markierung von Tumorzellen in Gewebe.

Aus dem Stand der Technik sind Markierungskörper bekannt, die in tumorzellenhaltige Bereiche in Gewebe eingebracht werden, um die Tumorzellen zu markieren. Die Markierungskörper bestehen in der Regel zumindest teilweise aus Metall, weshalb sie sich bei der Untersuchung durch bildgebende Verfahren von umliegenden Körperzellen abheben und so leicht detektiert werden können. Dadurch erleichtern sie das Wiederauffinden von befallenen Gewebestellen zu verschiedenen Therapiezeitpunkten, was sowohl für eine gezielte Behandlung betroffener Regionen als auch für die Erfolgskontrolle notwendig ist.

Üblich sind Markierungskörper aus einem mit einer Stahlummantelung versehenen Polymerkern oder reine Titan- oder Edelstahlkörper, die über eine Kanüle an die zu markierende Stelle befördert und dort freigesetzt werden. Diese weisen jedoch aufgrund ihres geringen Durchmessers keine gute Sichtbarkeit in bildgebenden Verfahren auf. Außerdem verbleiben sie häufig nicht an der vorgesehenen Stelle und bieten daher eine geringe Zuverlässigkeit. Andere Markierungskörper bestehen aus einem zu einer Spirale gewundenen Formgedächtnismetall, das nach der Platzierung im Gewebe eine Ringform annimmt. Diese bieten beim Blick auf den Ringumfang eine höhere Sichtbarkeit, die von den Seiten nicht gegeben ist, weshalb die Platzierung eines solchen Markierungskörpers größte Sorgfalt und Expertise des medizinischen Personals erfordert.

DE 10 2016 110 350 A1 beschreibt einen Markierungskörper zum Implantieren in ein Gewebe mit einer von mindestens einem elastischen Metalldraht gebildeten, komprimierbaren und selbstexpandierenden Tragstruktur, die in einem expandierten Zustand einen Innenraum einschließt, wobei der Markierungskörper ausgebildet ist, aus einem komprimierten Zustand, auch gegen einen an einer zu markierenden Gewebestelle herrschenden Gewebedruck, selbstständig in einen expandierten Zustand überzugehen, und der Markierungskörper im expandierten Zustand eine hohle, annähernd kugelförmige Gestalt aufweist. Aufgrund seiner aus Drähten gebildeten Struktur weist ein solcher Markierungskörper eine geringe Radialkraft bei der Expansion auf, was sich als problematisch bei festeren Gewebetypen erweisen kann.

Aus WO 2017/207777 A1 ist ein Markierungskörper zur Implantation in Gewebe bekannt, der eine selbstexpandierende Struktur aus einem Formgedächtnismetall aufweisen kann. Das Formgedächtnismetall kann dabei eine röhrenartige Struktur bilden, die entlang ihrer Länge längliche Öffnungen aufweist, die sich zwischen zwei Endabschnitten der röhrenartigen Struktur erstrecken. In einem Anwendungszustand liegt die röhrenartige Struktur gestaucht vor, sodass sich zwischen jeweils zwei benachbart zueinander vorliegenden länglichen Öffnungen verbleibende Streifen aus Formgedächtnismetall ausbauchen. Im Nicht-Anwendungszustand liegt die röhrenartige Struktur gestreckt vor.

Eine solche Struktur ist auch von anderen Implantaten, wie beispielsweise Embolisationsvorrichtungen, aus US 2014/0371778 A1 und DE 601 16 956 T2 bekannt.

Zur Verankerung eines Markierungskörpers oder anderer Implantate im Gewebe sind aus dem Stand der Technik unterschiedliche Lösungen bekannt. WO 2017/207777 A1 schlägt für eine Ausführungsform mit Drähten als Stützstruktur vor, einige Drahtenden über einen verschließenden Endabschnitt hinausragen zu lassen und hakenförmig umzubiegen. US 2014/0371778 A1 schlägt eine Collagenhülle zur verbesserten Haftung am Gewebe vor, und aus US 2007/0135826 A1 sind seitliche Widerhaken an Stützstreifen einer Implantatstruktur bekannt. US 2014/0046347 A1 lehrt die Verwendung von Ankern zur Befestigung medizinischer Vorrichtungen im Gewebe, wobei die Anker, die aus einem röhrenförmigen Element mit umbiegbaren Ankerarmen bestehen, in einem Hohlraum der medizinischen Vorrichtung vorgelegt werden und nach Implantation derselben durch entsprechend vorgesehene Öffnungen ausgefahren werden können, um die medizinischen Vorrichtung im Gewebe zu verankern.

Ausgehend von diesem Stand der Technik ist es Aufgabe der vorliegenden Erfindung, einen verbesserten Markierungskörper bereitzustellen.

Diese Aufgabe wird durch einen Markierungskörper mit den Merkmalen des Anspruchs 1 gelöst.

Die weitere Aufgabe, ein Verfahren bereitzustellen, um einen verbesserten Markierungskörpers in einem vereinfachten Fertigungsprozess herstellen zu können, wird durch das Verfahren mit den Merkmalen des unabhängigen Anspruchs 9 gelöst.

Ausführungsformen der erfindungsgemäßen Gegenstände sind in den jeweiligen Unteransprüchen ausgeführt.

Ein erfindungsgemäßer Markierungskörper, der zur Markierung von Tumorzellen in Gewebe ausgebildet ist, weist gemäß der vorliegenden Erfindung eine selbstexpandierende Struktur aus einem Formgedächtnismetall auf, wobei das Formgedächtnismetall eine röhrenartige Struktur bildet. Diese weist entlang ihrer Länge zumindest zwei längliche Öffnungen auf, die sich nicht weiter als von einem Kopfabschnitt zu einem Fußabschnitt der röhrenartigen Struktur erstrecken. In einem Anwendungszustand liegt die röhrenartige Struktur gestaucht vor, und es bauchen sich zwischen jeweils zwei benachbart zueinander vorliegenden länglichen Öffnungen verbleibende Streifen aus Formgedächtnismetall aus. In einem Nicht-Anwendungszustand liegt die röhrenartige Struktur gestreckt vor.

Unter selbstexpandierend wird hierin verstanden, dass das Formgedächtnismetall ohne Einfluss von äußeren Kräften in eine Form größeren Volumens übertritt.

Vorteilhaft ist der erfindungsgemäße Markierungskörper, der nicht aus Draht gefertigt ist, geeignet, hohen Expansionsdruck aufzubringen.

Als Anwendungszustand wird hier der Zustand bezeichnet, in dem sich der Markierungskörper befindet, wenn er keinen ihn verformenden Kräften ausgesetzt ist, die Struktur also selbst expandiert ist. Generell kann der Anwendungszustand ein Zustand sein, den der Markierungskörper annimmt, wenn er beispielsweise seine finale Position an einem zu markierenden Ort, etwa einem Gewebe, erreicht hat, um dort zu verbleiben. Demgegenüber wird als Nicht-Anwendungszustand der Zustand bezeichnet, in dem der Markierungskörper unter Einfluss äußerer Kräfte verformt ist. Dies kann beispielsweise ein Transportzustand sein, während dem der Markierungskörper bereits anwendungsbereit in der Kanüle vorliegt oder ein anderer Zustand, in dem der Markierungskörper bereitgelegt aber noch nicht anwendungsbereit ist.

Die röhrenartige Struktur gilt hierin als gestaucht, wenn sie ihrer Länge nach zusammengedrückt und dadurch gekürzt ist, und als gestreckt, wenn diese Verkürzung unter Einfluss äußerer Kräfte wieder zurückgeformt ist.

An dieser Ausführungsform ist vorteilhaft, dass aus einem Formgedächtnismetall ein Markierungskörper hergestellt werden kann, der im Anwendungszustand voluminös ausgebaucht ist. Durch die Ausbauchung der Streifen erfährt der Markierungskörper eine Umfangserweiterung, was vorteilhaft seine Sichtbarkeit und damit Wiederauffindbarkeit in bildgebenden Verfahren, insbesondere bei Röntgen- und Ultraschalluntersuchungen, erhöht. Da Ultraschalluntersuchungen für den menschlichen Körper weitaus weniger belastend sind als röntgenologische Verfahren, ist eine erhöhte Sichtbarkeit hier besonders vorteilhaft.

Beim Übergang in den Anwendungszustand übt der erfindungsgemäße Markierungskörper über die sich ausbauchenden Streifen eine höhere Radialkraft auf seine Umgebung aus, als es bei bekannten Markierungskörpern der Fall ist, wodurch die selbstexpandierende Struktur aus Formgedächtnismetall sich auch in Umgebungen mit höherer Festigkeit vollständig expandieren kann.

Durch die Verwendung des Formgedächtnismetalls kann die für den Anwendungszustand gewünschte Struktur bei ihrer Fertigung definiert werden. Für den Transport und während eines Implantationsvorgangs in Gewebe etwa liegt die röhrenartige Struktur aber in gestreckter Form vor, was vorteilhaft einen geringeren Platzbedarf und einen besseren, reibungsärmeren Transport durch eine für das Einbringen in Gewebe benötigte Kanüle bedeutet.

Erfindungsgemäß weist der Markierungskörper ferner Ankermittel auf. Zumindest ein Streifen hat zumindest ein Ankermittel entlang der Umfangsrichtung und/oder entlang seiner Länge. Das Ankermittel wird durch eine Zunge gebildet, die durch eine(n) oder mehrere Einschnitt(e) oder Ausnehmung(en) bereitgestellt wird, der/die in den Streifen eingebracht ist/sind. Dabei weist die Zunge eine Wurzel auf, über die sie radial bewegbar mit dem Streifen verbunden ist. Die Wurzel jeder der Zungen schließt an den Kopfabschnitt oder den Fußabschnitt an oder liegt dazu in Längsrichtung versetzt.

Unter bewegbar ist hierbei zu verstehen, dass die Zunge beim Übergang vom Nicht-Anwendungszustand in den Anwendungszustand bewegt wird, und zwar kann die freigelegte Zunge sich ausgehend von ihrem Kopfende über die Ausbauchung der einzelnen Streifen hinaus weiter in radialer Richtung aufbiegen und sich dabei krümmen. So weist die Zunge mit ihrem Kopfende im Anwendungszustand in das umliegende Gewebe hinein und bildet vorteilhaft ein Ankermittel, das, vergleichbar mit einem Haken, den Markierungskörper an seiner Position in der Umgebung fixieren kann, was sonst lediglich über den Anpressdruck des Markierungskörpers auf seine Umgebung geschieht. Außerdem vorteilhaft ist an dieser Ausführungsform, dass die Ankermittel sich ebenfalls aus der röhrenartigen Struktur erzeugen lassen, wodurch die glatte Außenkontur des Markierungskörpers erhalten bleibt.

Wenn mehrere Streifen Zungen aufweisen, ist es vorteilhaft möglich, die Zungen beispielsweise drehsymmetrisch anzuordnen, sodass sich der Markierungskörper in mehreren Richtungen im umliegenden Gewebe verankern kann, wodurch die Zuverlässigkeit der Verankerung erhöht wird. Außerdem wird so das Bereitlegen des Markierungskörpers in der Kanüle vereinfacht, da dieser nicht in einer vorgegebenen rotatorischen Ausrichtung vorliegen oder in das Gewebe eingebracht werden muss. Eine bevorzugte Ausführungsform besitzt an jedem Streifen eine Zunge.

Liegen die Wurzeln der Zungen zu Kopfabschnitt oder Fußabschnitt in Längsrichtung versetzt an, sind die Zungen kürzer und damit stabiler und auch die mit Zungen versehenen Streifen weisen im Bereich der Zungenwurzel mehr Stabilität auf als bei nicht versetzten Wurzeln.

Eine Anordnung von mehreren Zungen an einem Streifen bietet vorteilhaft eine höhere Flexibilität der Ausgestaltung der Verankerung sowie von verschiedenen wiedererkennbaren Designs.

In einer weiteren Ausführungsform des erfindungsgemäßen Markierungskörpers ist der Kopfabschnitt der röhrenartigen Struktur ein Kopfbund und der Fußabschnitt ein Fußbund. Kopf- bzw. Fußbund meint hierin, dass ein Zusammenhalt der Streifen zumindest an einem solchen Bund gegeben ist. Dadurch nimmt der Markierungskörper im Anwendungszustand annähernd ellipsoide Strukturen an. Die länglichen Öffnungen in dem Markierungskörper können wenigstens ein Stück weit oder ganz parallel zueinander verlaufen; sie können zwischen dem Kopfabschnitt und dem Fußabschnitt axial oder helixartig verlaufende Einschnitte und/oder Ausnehmungen sein.

Nach einer weiteren Ausführungsform des erfindungsgemäßen Markierungskörpers ist das Formgedächtnismetall des Markierungskörpers Nitinol. Nitinol meint hier eine Nickel-Titan-Legierung, wobei der Name ein Akronym für Nickel Titanium Naval Ordnance Laboratory ist. Es ist mit seinen superelastischen Eigenschaften für den Einsatz in medizintechnischen Produkten geeignet und mit bekannten Verarbeitungsverfahren zu verarbeiten. Da Nitinol ähnliche biomechanische Eigenschaften wie menschliches Gewebe aufweist, kann es den Beanspruchungen, denen es im Anwendungszustand ausgesetzt ist, Stand halten. Des Weiteren ist Nitinol vorteilhaft ein biokompatibles Material, das bei Kontakt mit Körperzellen keine Abwehrreaktionen hervorruft.

Die röhrenartige Formgedächtnisstruktur kann ein zu einem Rohr gebogenes Blech, das längsseits geschlossen ist oder das längsseits noch einen Öffnungsschlitz aufweist, oder ein Rohr sein. Durch die Fertigung aus einem einzigen Blech, Rohr oder Röhrchenstück ergibt sich eine einerseits kostengünstige Fertigung und andererseits besonders gut handhabbare Form, da durch die glatte Außenkontur der Markierungskörper leicht zu reinigen und sehr gut in einer Kanüle handhabbar ist.

Öffnungstypen der zwischen dem Kopfabschnitt und dem Fußabschnitt verlaufenden axialen oder helixartigen Öffnungen können auch kombiniert werden.

Helixartig meint, dass die Schnitte oder Ausnehmungen sich um die Mantelfläche der röhrenartigen Struktur winden, wobei die Zahl der umlaufenen Umfänge beliebig ist und auch kleiner als eins sein kann. Dabei wird durch die Anzahl der Einschnitte und Ausnehmungen sowie deren Abstand voneinander zugleich die Streifenanzahl und -breite festgelegt. Einschnitte stellen die einfachste und damit kostengünstigste Variante für die länglichen Öffnungen dar. Durch Ausnehmungen mit Materialabtrag oder eine Kombination aus Schnitten und Ausnehmungen können eine Vielzahl unterschiedlicher Designs erzeugt werden. Diese können vorteilhaft verschiedene Körperregionen mit in bildgebenden Verfahren unterscheidbar wiederzuerkennenden Designs markieren.

Nach einer weiteren Ausführungsform kann zumindest eine Zunge an ihrem Kopfende mehrere Enden, bevorzugt Spitzen oder abgerundete Spitzen, aufweisen. Bevorzugt sind der oder die Einschnitte U-förmig oder W-förmig. Abgerundete Spitzen bilden gewebefreundliche Verankerungsstellen. Zungen mit einer Spitze bzw. abgerundeten Spitze, wie sie durch V- oder bevorzugt U-förmige Einschnitte erzeugt werden, sind für eine einfache Verankerung ausreichend und vorteilhaft, da sie sehr einfach erzeugt werden können. Durch W-förmige Einschnitte werden Zungen mit zwei Spitzen erzeugt. Dies erhöht die Wahrscheinlichkeit, dass sich zumindest eine Spitze auf die vorgesehene Weise verankert. Je nach Ausführung kann das dazwischenliegende Material entfernt sein, oder es ist noch mit dem Rohr verbunden und bildet seinerseits eine Zunge, die in die entgegengesetzte Richtung bewegbar ist. Solch eine Kombination von sich gegenüberliegenden Zungen kann vorteilhaft die Zuverlässigkeit der Verankerung erhöhen. Es sind weitere Einschnittformen denkbar, die ein, zwei oder mehr Enden erzeugen, z.B. nicht geschlossene, trapezförmige Einschnitte.

Des Weiteren kann vorgesehen sein, dass der Kopfabschnitt oder der Fußabschnitt der röhrenartigen Struktur, oder beide, mit einem Verschlusselement versehen ist bzw. sind. Das Verschlusselement ist bevorzugt eine Kappe und erhöht vorteilhaft die Sichtbarkeit des Markierungskörpers in bildgebenden Verfahren, wenn die Blickrichtung axial zu der röhrenartigen Struktur verläuft.

Gemäß noch einer weiteren Ausführungsform des erfindungsgemäßen Markierungskörpers ist in der röhrenartigen Struktur ein länglicher Körper angeordnet, bevorzugt ein Stift oder ein Röhrchen, der ein Material, ausgewählt aus der Gruppe umfassend ferromagnetische Metalle und Legierungen, Formgedächtnismetalle und Polymere, bevorzugt leitfähige Polymere, oder Kombinationen der Vorgenannten, aufweist. Ein solcher Körper erhöht die Sichtbarkeit in axialer Blickrichtung ebenfalls. Außerdem kann er vorteilhaft als dotiertes Polymer ausgebildet sein, das in einem bildgebenden Verfahren ein charakteristisches Bild aufweist und damit die Unterscheidbarkeit verschiedener an unterschiedlichen Stellen angebrachter Markierungskörper ermöglicht. Ist der eingelegte Körper magnetisch, so bietet er vorteilhaft die zusätzliche Möglichkeit der Detektion des platzierten Markierungskörpers über eine Analyse des Magnetfeldes.

Es kann weiter vorgesehen sein, dass der Markierungskörper eine Umhausung aus einer hydrophilen, quellbaren Substanz, bevorzugt aus einem Hydro-Gel oder einem Kollagen, aufweist, wobei die selbstexpandierende Struktur aus dem Formgedächtnismetall im komprimierten Zustand, d. h. im Nicht-Anwendungszustand in der Umhausung vorliegt.

Die Umhausung ist hierin als gefüllte Umhausung zu verstehen, die gleichsam ein Bett mit einer definierten Außenkontur für den komprimierten, umfassten Markierungskörper bildet. Dabei kann der Innenraum des Markierungskörpers ganz oder teilweise gefüllt sein. Unter Kontakt mit einem wasserhaltigen Medium, das beispielsweise eine Gewebeflüssigkeit sein kann, wenn der Markierungskörper in menschliches Gewebe eingesetzt wird, quillt eine solche - zunächst in einem trockenen Zustand vorliegende - Umhausung auf und vergrößert ihr Volumen auf ein Vielfaches. Dadurch wird vorteilhaft die Expansion des Markierungskörpers unterstützt. Bei der Ausführung des Markierungskörpers in einer bevorzugten Ausführungsform aus Nitinol kann dieses aufgrund seiner superelastischen Materialeigenschaften besonders stark expandieren und somit kann die Volumenerweiterung besonders vorteilhaft auf den Markierungskörper übertragen werden.

In einer weiteren Ausführungsform hat die röhrenartige Struktur im Anwendungszustand an ihrer am weitesten ausgebauchten Stelle einen Durchmesser im Bereich von 2 mm bis 15 mm, bevorzugt im Bereich von 3 mm bis 8 mm, und eine Länge im Bereich von 2 mm bis 15 mm, bevorzugt im Bereich von 4 mm bis 9 mm. Diese Abmessungen bedeuten vorteilhaft, dass der Markierungskörper im Nicht-Anwendungszustand ausreichend klein ist, um in einer Kanüle bereitgelegt und mit Methoden der minimalinvasiven Chirurgie in Gewebe eingesetzt zu werden und im Anwendungszustand ausreichend groß, um eine hohe Wiederauffindbarkeit der markierten Stelle bei der Anwendung von bildgebenden Verfahren sicherzustellen.

Ein erfindungsgemäßes Verfahren zur Herstellung eines erfindungsgemäßen Markierungskörpers umfasst folgende Schritte:
a) Bereitstellen eines Halbzeugs in Form eines Rohrs oder eines Blechs aus einem Formgedächtnismetall,
b) Einbringen der zumindest zwei länglichen Öffnungen entlang der Längsseite des Halbzeugs zwischen Kopfabschnitt und Fußabschnitt, wobei zwischen jeweils zwei benachbart zueinander vorliegenden länglichen Öffnungen Streifen gebildet werden, und nach Schritt
b) Herstellen von zumindest einer Zunge als Ankermittel durch Einbringen von Schnitten oder Ausnehmungen in zumindest einen der Streifen, und, falls das Halbzeug in Form eines Blechs vorliegt, b') Biegen des Blechs entlang seiner Längsachse, bis es eine röhrenartige Struktur aufweist,
c) mittels eines Werkzeugs Angreifen an Kopfabschnitt und Fußabschnitt und Aufbringen einer axialen Kraft und dabei Stauchen des Halbzeugs, und als Schritt c') Aufbiegen der Zunge, die über eine Wurzel mit dem Streifen verbunden ist, in radialer Richtung nicht weiter als bis zur Wurzel,
d) Erhitzen des Halbzeugs auf eine Solltemperatur und Halten der Solltemperatur für eine Solldauer, dabei dem Halbzeug Aufprägen der Form des Halbzeugs während der Stauchung,
e) Erkaltenlassen des Halbzeugs.

Das Halbzeug kann also ein Rohr oder ein Blech, das zu einer röhrenartigen Struktur gebogen wird, aus Nitinol oder einem anderen Formgedächtnismetall sein. Die Fertigung geht somit von einem einzelnen Werkstück aus und ist damit einfach und kostengünstig durchführbar.

Die Öffnungen können beispielsweise über Laserschneiden, ein insbesondere für Nitinol bevorzugtes und geeignetes Verfahren, in das Halbzeug eingebracht werden. Es sind jedoch auch andere für das jeweils verwendete Formgedächtnismetall geeignete Bearbeitungsverfahren möglich, zum Beispiel (nicht abschließende Aufzählung) Wasserstrahlschneiden oder Drahterodieren.

Die Solltemperatur, um das Formgedächtnis zu aktivieren, liegt üblicherweise im Bereich von 400 bis 650 Grad Celsius und wird bevorzugt für eine Solldauer nicht über drei Minuten gehalten, besonders bevorzugt nicht über einer Minute. Kürzere Haltedauern beschleunigen bekanntermaßen die Fertigung und sind damit kostensparend.

Das Erhitzen kann extern, etwa durch Beheizen über das Werkzeug, oder intern, beispielsweise durch induktive Erwärmung oder Anlegen einer elektrischen Spannung an Kopf- und Fußabschnitt des Halbzeugs, das sich durch einen elektrischen Strom entgegenstehenden Widerständen erwärmt, geschehen.

Mit diesem Verfahren können erfindungsgemäße Markierungskörper gefertigt werden, die Ankermittel, insbesondere Zungen, zur Verankerung des Markierungskörpers an der zu markierenden Stelle aufweisen. Die Ankermittel werden vor dem Schritt des Erhitzens d) in Anwendungsposition gebracht, damit diese Form aufgeprägt wird.

Eine Verwendung eines erfindungsgemäßen Markierungskörpers sieht die Verwendung als Marker für Tumorzellen in einem Gewebe vor. Dafür wird der Markierungskörper gefertigt und im Nicht-Anwendungszustand in einer Kanüle bereitgelegt. Unter Verwendung eines bildgebenden Verfahrens wird dann eine Stelle des menschlichen Körpers, die von Tumorzellen befallen ist, lokalisiert und die Kanüle so platziert, dass der Markierungskörper durch die Kanüle geschoben werden kann und an der vorgesehenen Stelle aus der Kanüle austritt. Beim Austritt aus der Kanüle geht der erfindungsgemäße Markierungskörper in den Anwendungszustand über, indem die selbstexpandierende Struktur aus Formgedächtnismetall expandiert. Durch die Volumenerweiterung wirkt der Markierungskörper einen Anpressdruck auf das ihn umgebende Gewebe aus und setzt sich dadurch dort fest. Weist der Markierungskörper Ankermittel auf, gehen diese ebenfalls beim Übergang in den Anwendungszustand in eine aufgebogene Stellung über, sodass sie den Markierungskörper zusätzlich verankern können. Somit ist die Körperstelle, an die der Markierungskörper platziert ist, derart markiert, dass sie unter Verwendung eines bildgebenden Verfahrens, insbesondere Ultraschall, wieder aufgefunden werden kann, da der Markierungskörper unter diesen bildgebenden Verfahren besonders gut sichtbar ist. Diese Sichtbarkeit bleibt erhalten, auch wenn die Tumorzellen selbst aufgrund eines angewendeten Therapieverfahrens gar nicht mehr oder nicht mehr in so großer Menge wie zuvor vorhanden sind und die markierte Stelle daher über eine Lokalisation von Tumorzellen nicht mehr eindeutig bestimmt werden kann. Die Verwendung selbst ist nicht Teil der vorliegenden Erfindung.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig. 1**: eine perspektivische Ansicht eines Markierungskörper aus einem Blech
- **Fig. 2a**: eine perspektivische Ansicht eines Markierungskörpers mit Ankermitteln
- **Fig. 2b**: eine Längsansicht des Markierungskörpers aus Fig. 2a,
- **Fig. 2c**: eine Frontansicht des Markierungskörpers aus Fig. 2a,
- **Fig. 3**: einen Markierungskörper gem. Fig. 2a,b,c mit Umhausung
- **Fig. 4a**: einen Markierungskörper mit Ankermitteln mit abgerundeten Spitzen
- **Fig. 4b**: den Markierungskörper aus Fig. 4a mit Kappen
- **Fig. 4c**: den Markierungskörper aus Fig. 4a mit eingebrachtem Stift
- **Fig. 5**: einen Markierungskörper mit teilweise geöffnetem Kopf- und Fußabschnitt
- **Fig. 6a**: einen Markierungskörper mit helixartigen Schnitten
- **Fig. 6b**: den Markierungskörper aus Fig. 6a im Nicht-Anwendungszustand

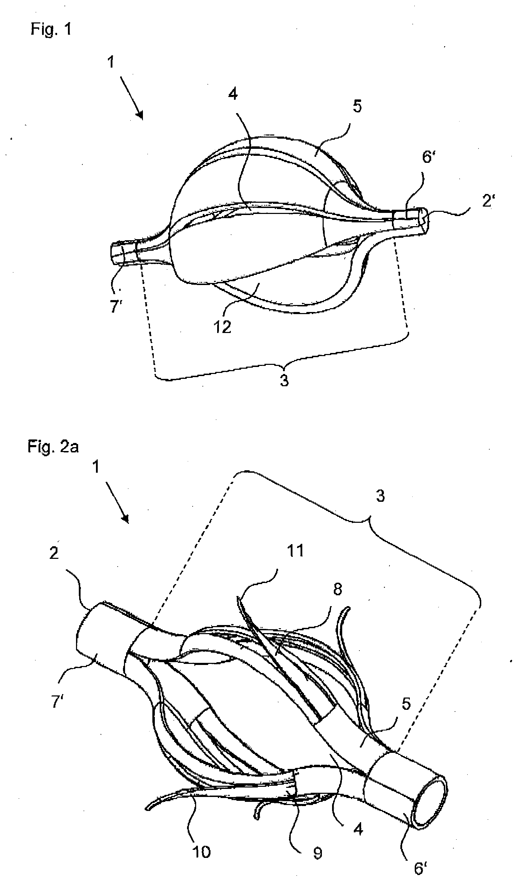

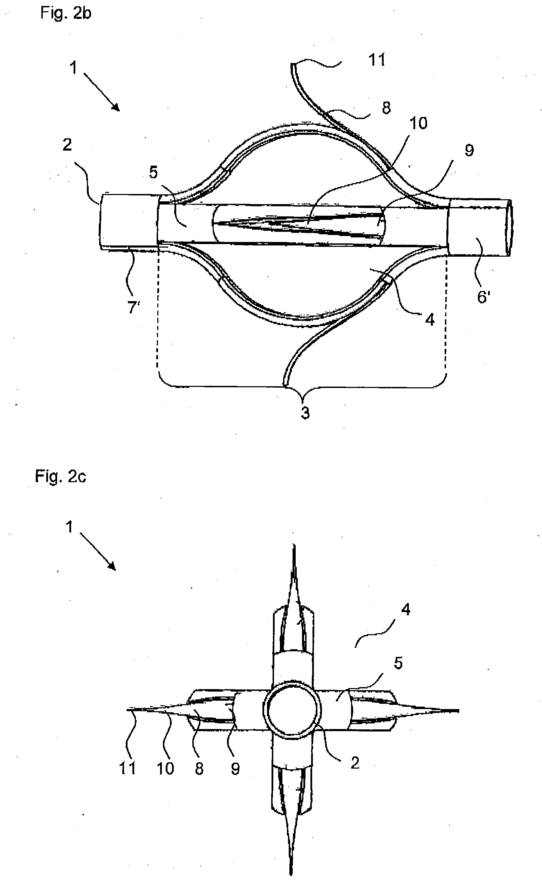

Die erfindungsgemäße Vorrichtung bezieht sich auf einen Markierungskörper mit Ankermitteln, ausgebildet zur Markierung von Tumorzellen in Gewebe, wie in **Fig. 2 bis 5** gezeigt.

**Fig. 1** zeigt einen Markierungskörper 1 im Anwendungszustand. Er weist also diejenige Formgedächtnisstruktur 3 auf, die er auch aufweist, wenn er beispielsweise in ein Gewebe eingebracht ist. Er besteht aus einem zum Rohr gebogenen Blech 2' aus einem Formgedächtnismetall, welches längsseitig noch einen Öffnungsschlitz 12 aufweist. Außerdem weist er sechs längliche Öffnungen 4 auf, die hier Schnitte sind, die zwischen Kopfbund 6' und Fußbund 7' verlaufen, und somit auch sechs Streifen 5, die dazwischen verlaufen. Diese bilden die hier dargestellte ausgebauchte Formgedächtnisstruktur 3, die ein vergrößertes Volumen und eine durch die breiten Streifen 5 hohe Sichtbarkeit in verschiedenen Blickrichtungen bei der Betrachtung mittels eines bildgebenden Verfahrens bietet. Über die Streifen 5 wirkt eine radiale Kraft auf die Umgebung des Markierungskörpers 1, der sich so dort festsetzt. Nicht in den Figuren dargestellt ist der zugehörige Nicht-Anwendungszustand des Markierungskörpers 1, der dann gestreckt und somit nicht ausgebaucht vorliegt, und aufgrund seiner röhrenartigen Struktur leicht in einer Kanüle vorgeladen und in dieser bewegt werden kann.

Grundsätzlich ist zu sagen, dass die Anzahl der längliche Öffnungen 4 bei allen erfindungsgemäßen Markierungskörpern 1 eher bei mindestens 3, 4, 5, 6 oder gegebenenfalls sogar mehr, bis zu 14, liegen kann, um eine ideal kugelige Form des zwischen Kopf- und Fußabschnitt 5, 6 liegenden Bereichs im Anwendungszustand zu erlangen. Es dürfen lediglich nicht so viele Öffnungen oder Einschnitte sein, dass die Struktur instabil oder gar drahtartig wird.

In **Fig. 2a bis 2c** ist ein erfindungsgemäßer Markierungskörper 1 mit Ankermitteln im Anwendungszustand dargestellt, wobei die Ankermittel Zungen 8 sind. Der Markierungskörper ist hier erfindungsgemäß aus einem Rohr 2 aus einem Formgedächtnismetall gefertigt. Es weist vier längliche Öffnungen 4 auf, die hier in Form von vier parallelen Schnitten entlang der Länge des Rohrs 2 vorliegen. Die so gebildeten vier Streifen 5 verlaufen zwischen Kopfbund 6' und Fußbund 7' (in Fig. 2c nicht sichtbar), da die Schnitte dort beginnen bzw. enden. Im in **Fig. 2a bis 2c** dargestellten Anwendungszustand sind die Streifen zwischen Kopf- 6' und Fußbund 7' ausgebaucht und bilden so das Gerüst der Formgedächtnisstruktur.

In der dargestellten Ausführungsform ist jeder Streifen 5 V-förmig eingeschnitten, wobei die V-förmigen Einschnitte weder den Kopfbund 6' noch den Fußbund 7' noch die Öffnungen 4 zwischen den Streifen 5 tangieren. So wird in jedem Streifen 5 eine Zunge 8 als Ankermittel freigelegt. Jede Zunge 8 weist an ihrem Kopfende 10 eine Spitze 11 auf. Die Spitzen weisen von der Formgedächtnisstruktur 3 weg in die Umgebung hinein, wie in **Fig. 2b und 2c** besonders gut zu sehen ist. Wenn der Markierungskörper 1 beispielsweise in einer von Tumorzellen befallenen Gewebestelle platziert ist, kann er über diese Spitzen an vier Punkten im umliegenden Gewebe zusätzlich fixiert werden. Die Zungen 8 sind über ihre Wurzeln 9 mit dem Streifen 5, in den sie hineingeschnitten sind, verbunden. Die Wurzeln 9 liegen zum Kopfbund 6' gewandt in Richtung des Fußbunds 7' versetzt.

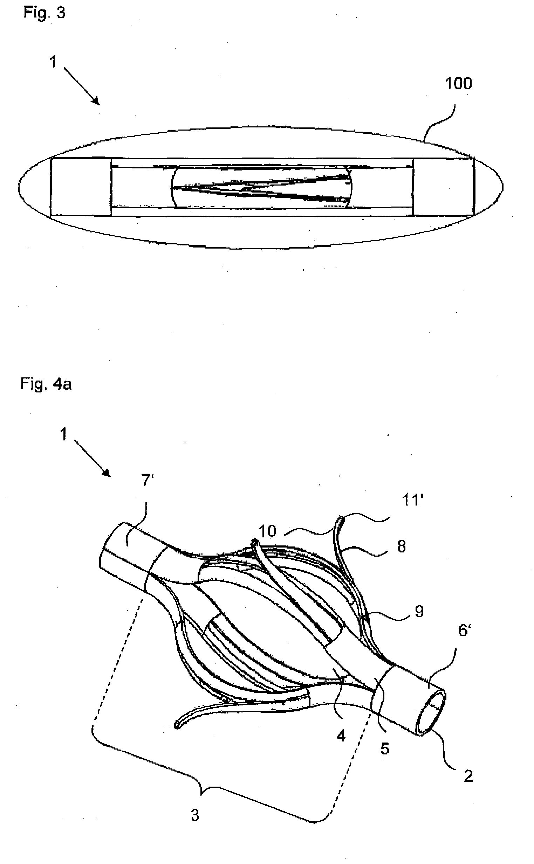

In **Fig. 3** ist ein Markierungskörper 1 mit Umhausung 100 dargestellt, der sich im Nicht-Anwendungszustand befindet und somit röhrenförmig ist. Das Material, welches die Umhausung bildet, ist beispielsweise ein Hydro-Gel und liegt in und um den Markierungskörper in einem trockenen Zustand vor. In diesem Zustand kann der Markierungskörper an die zu markierende Stelle gebracht werden. Nicht dargestellt ist, dass das Hydro-Gel bei Kontakt mit Flüssigkeit aufquillt, was das Entfalten der Formgedächtnisstruktur unterstützt und damit der gesamte Markierungskörper sein Volumen deutlich erweitert.

**Fig. 4a** zeigt einen Markierungskörper 1, der ähnlich aufgebaut ist wie der Markierungskörper 1 in **Fig. 2****.** Er weist vier längliche Öffnungen 4 und vier Streifen 5 auf, wobei in jedem Streifen 5 jeweils eine Zunge 8 eingebracht ist. Im Unterschied zum Markierungskörper in **Fig**. **2** sind jedoch in dieser Ausführungsform die Zungen durch U-förmige Einschnitte erzeugt, sodass die Spitzen 11' der Zungen 8 abgerundet sind, was im direkten Vergleich der **Fig. 2a** und **Fig. 4a** besonders deutlich wird.

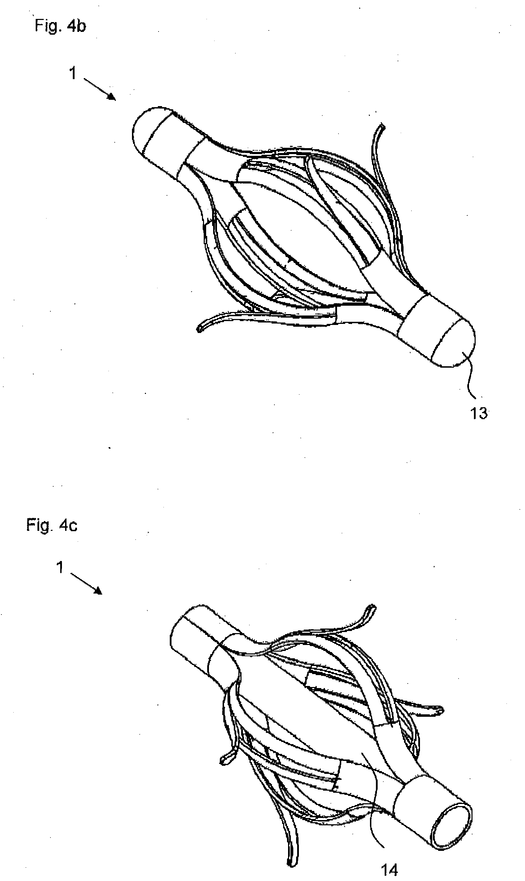

Der in **Fig. 4b** gezeigte Markierungskörper 1 entspricht demjenigen aus Fig. 4a, erweist aber als zusätzliches Merkmal Kappen 13 auf, die an Kopfbund 6' und Fußbund 7' als Verschlusselemente angebracht sind. Den Markierungskörper 1 aus **Fig. 4a****,** der in seinem Inneren einen Stift aufweist, zeigt **Fig. 4c****.** Beides sind Ausführungsvarianten, die die Sichtbarkeit der Markierungskörper 1 erhöhen und Gestaltungsvarianten für das im bildgebenden Verfahren sichtbare Bild der Markierungskörper 1 darstellen.

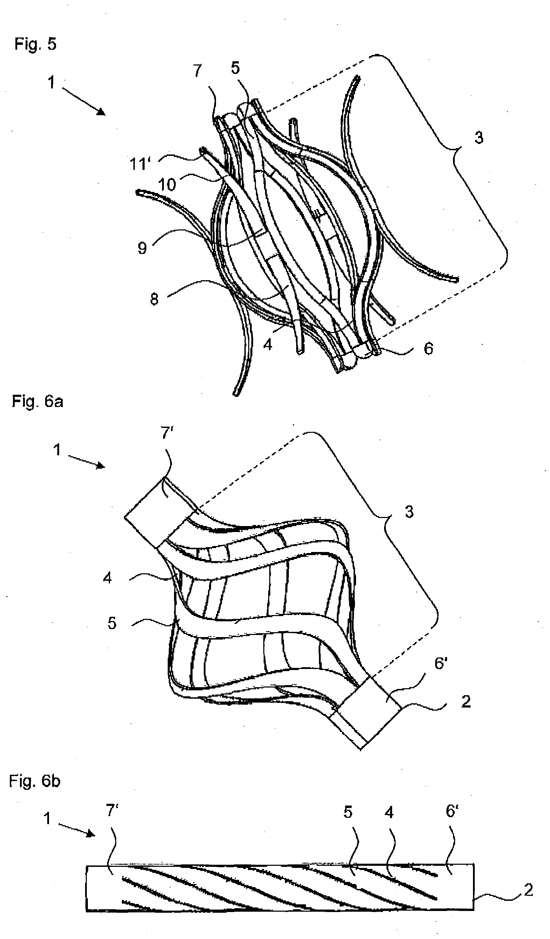

In **Fig. 5** ist ein erfindungsgemäßer Markierungskörper zu sehen, der neben vier axialen Schnitten 4 weitere Ausnehmungen und Schnitte aufweist. Oberhalb und unterhalb der Öffnungen 4 wird jeweils ein Kopfabschnitt 6 und Fußabschnitt 7 stehen gelassen. Dabei verlaufen auch innerhalb von Kopfabschnitt 6 und Fußabschnitt 7 Ausnehmungen. An vier Streifen 5 sind je zwei U-förmige Einschnitte eingebracht, die Ankermittel bilden. An jedem der Streifen 5 ist je ein Paar von sich gegenüberliegenden Zungen 8 angeordnet, die mit ihren abgerundeten Spitzen 11' voneinander weg weisen. Durch die symmetrische Anordnung der Zungen kann das Wegbewegen des Markierungskörpers 1 in verschiedenen Richtungen verhindert werden.

Fig. **6a** und **6b** zeigen einen Markierungskörper 1, der aus einem Rohr 2 gefertigt ist, in das sechs helixartig zwischen Kopfbund 6' und Fußbund 7' verlaufende Schnitte 4 eingebracht sind, die sich etwas mehr als einmal um den Umfang des Rohrs 2 winden, darüber hinaus aber keine weiteren Einschnitte oder Ausnehmungen. Dabei zeigt **Fig. 6b** den Markierungskörper im Nicht-Anwendungszustand. Die röhrenartige Struktur, die besonders leicht und reibungsarm durch eine Kanüle transportiert werden kann, wird hier deutlich. Im Anwendungszustand dieses Markierungskörpers 1, der in **Fig. 6a** zu sehen ist, liegt die expandierte Formgedächtnisstruktur 3 vor, die durch die sechs ausgebauchten Streifen 6 gebildet wird. Die Figuren sind nur schematische Darstellungen, insbesondere sind die Größenverhältnisse zwischen dem Anwendungs- und Nicht-Anwendungszustand nicht in den Figuren wiedergegeben.

### BEZUGSZEICHENLISTE

- 1: Markierungskörper
- 2, 2': Rohr, Blech
- 3: Formgedächtnisstruktur
- 4: längliche Öffnung
- 5: Streifen
- 6, 6': Kopfabschnitt, Kopfbund
- 7, 7': Fußabschnitt, Fußbund
- 8: Zunge
- 9: Wurzel
- 10: Kopfbereich
- 11, 11': Spitze, abgerundete Spitze
- 12: Öffnungsschlitz
- 13: Kappe
- 14: Stift
- 100: Umhausung

## Patentansprüche

1. Markierungskörper (1), ausgebildet zur Markierung von Tumorzellen in Gewebe, der eine selbstexpandierende Struktur (3) aus einem Formgedächtnismetall aufweist, wobei das Formgedächtnismetall eine röhrenartige Struktur bildet, die entlang ihrer Länge zumindest zwei längliche Öffnungen (4) aufweist, die sich nicht weiter als von einem Kopfabschnitt (6) zu einem Fußabschnitt (7) der röhrenartigen Struktur erstrecken,
wobei
- in einem Anwendungszustand die röhrenartige Struktur gestaucht vorliegt, und sich zwischen jeweils zwei benachbart zueinander vorliegenden länglichen Öffnungen (4) verbleibende Streifen (5) aus Formgedächtnismetall ausbauchen, und
- in einem Nicht-Anwendungszustand die röhrenartige Struktur gestreckt vorliegt, wobei der Markierungskörper Ankermittel aufweist,
**dadurch gekennzeichnet, dass**
zumindest einer der Streifen (5) zumindest ein Ankermittel entlang der Umfangsrichtung und/oder entlang seiner Länge aufweist,
und wobei das Ankermittel durch eine Zunge (8) gebildet wird, die durch einen oder mehrere Einschnitte oder Ausnehmungen bereitgestellt wird, der/die in den Streifen (5) eingebracht ist/sind,
wobei die Zunge (8) eine Wurzel (9) aufweist, über die sie radial bewegbar mit dem Streifen (5) verbunden ist, und wobei die Wurzel (9) jeder der Zungen (8)
- an den Kopfabschnitt (6) oder den Fußabschnitt (7) anschließt oder
- zu dem Kopfabschnitt (6) oder dem Fußabschnitt (7) in Längsrichtung versetzt liegt.

2. Markierungskörper (1) nach Anspruch 1,
**dadurch gekennzeichnet, dass**
der Kopfabschnitt (6) ein Kopfbund (6') und der Fußabschnitt (7) ein Fußbund (7') ist, und/oder wobei
die länglichen Öffnungen (4) zumindest teilweise parallel zueinander verlaufen und/oder die Öffnungen (4) zwischen dem Kopfabschnitt (6) und dem Fußabschnitt (7) axial oder helixartig verlaufende Einschnitte und/oder Ausnehmungen sind.

3. Markierungskörper (1) nach Anspruch 1 oder 2,
**dadurch gekennzeichnet, dass**
das Formgedächtnismetall Nitinol ist und/oder
die röhrenartige Formgedächtnisstruktur ein
- zu einem Rohr (2) gebogenes Blech (2'), das längsseits geschlossen ist,
- zu einem Rohr (2) gebogenes Blech (2'), das längsseits noch einen Öffnungsschlitz (12) aufweist,
- ein Rohr (2)
ist.

4. Markierungskörper (1) nach zumindest einem der Ansprüche 1 bis 3,
**dadurch gekennzeichnet, dass**, wenn die zumindest eine Zunge durch den/die Einschnitt/e bereitgestellt wird, der oder die Einschnitte U-förmig oder W-förmig sind und die zumindest eine Zunge (8) einen Kopfbereich (10) aufweist, wobei die Zunge an diesem Kopfbereich (10) mehrere Enden, bevorzugt Spitzen (11) oder abgerundete Spitzen (11'), aufweist.

5. Markierungskörper (1) nach zumindest einem der Ansprüche 1 bis 4,
**dadurch gekennzeichnet, dass**
der Kopfabschnitt (6) und/oder der Fußabschnitt (7) der röhrenartigen Struktur mit einem Verschlusselement versehen ist/sind, bevorzugt mit einer Kappe (13).

6. Markierungskörper (1) nach zumindest einem der Ansprüche 1 bis 5,
**dadurch gekennzeichnet, dass**
in der röhrenartigen Struktur ein länglicher Körper angeordnet ist, bevorzugt ein Stift oder ein Röhrchen,
der ein Material, ausgewählt aus der Gruppe umfassend ferromagnetische Metalle und Legierungen, Formgedächtnismetalle und Polymere, bevorzugt leitfähige Polymere, oder Kombinationen der Vorgenannten, aufweist.

7. Markierungskörper (1) nach zumindest einem der Ansprüche 1 bis 6,
**dadurch gekennzeichnet, dass**
der Markierungskörper (1) eine Umhausung (100) aus einer hydrophilen, quellbaren Substanz, bevorzugt aus einem Hydro-Gel oder einem Kollagen, aufweist, wobei die selbstexpandierende Struktur (3) aus dem Formgedächtnismetall im Nicht-Anwendungszustand in der Umhausung (100) vorliegt.

8. Markierungskörper (1) nach zumindest einem der Ansprüche 1 bis 7,
**dadurch gekennzeichnet, dass**
die röhrenartige Struktur im Anwendungszustand an ihrer am weitesten ausgebauchten Stelle einen Durchmesser im Bereich von 2 mm bis 15 mm, bevorzugt im Bereich von 3 mm bis 8 mm, und eine Länge im Bereich von 2 mm bis 15 mm, bevorzugt im Bereich von 4 mm bis 9 mm, aufweist.

9. Verfahren zur Herstellung eines Markierungskörpers (1) nach zumindest einem der Ansprüche 1 bis 8,
**umfassend die Schritte**
a) Bereitstellen eines Halbzeugs in Form eines Rohrs (2) oder in Form eines Blechs (2') aus einem Formgedächtnismetall,
b) Einbringen der zumindest zwei länglichen Öffnungen (4) entlang der Längsseite des Halbzeugs zwischen Kopfabschnitt (6) und Fußabschnitt (7), wobei zwischen jeweils zwei benachbart zueinander vorliegenden länglichen Öffnungen (4) Streifen (5) gebildet werden, und nach Schritt b) Herstellen von zumindest einer Zunge (8) als Ankermittel durch Einbringen von Schnitten oder Ausnehmungen in zumindest einen der Streifen (5), und, falls das Halbzeug in Form eines Blechs vorliegt, b') Biegen des Blechs (2') entlang seiner Längsachse, bis es eine röhrenartige Struktur aufweist,
c) mittels eines Werkzeugs Angreifen an Kopfabschnitt (6) und Fußabschnitt (7) und Aufbringen einer axialen Kraft und dabei Stauchen des Halbzeugs, und als Schritt c') Aufbiegen der Zunge (8), die über eine Wurzel (9) mit dem Streifen (5) verbunden ist, in radialer Richtung nicht weiter als bis zur Wurzel (9),
d) Erhitzen des Halbzeugs auf eine Solltemperatur und Halten der Solltemperatur für eine Solldauer, dabei dem Halbzeug Aufprägen der Form des Halbzeugs während der Stauchung,
e) Erkaltenlassen des Halbzeugs.

## Claims

1. A marking body (1), formed for marking tumour cells in tissue,
comprising a self-expanding structure (3) of a shape-memory metal, wherein
the shape-memory metal forms a tubular structure that has at least two elongated openings (4) along its length that extend no further than from a head section (6) to a foot section (7) of the tubular structure,
wherein
- the tubular structure is present compressed in an application condition and remaining strikes (5) of shape-memory metal protrude between two adjacent elongated openings (4) each, and
- the tubular structure is present stretched in a non-application condition,
wherein the marking body has anchoring means,
**characterised in that**
at least one strip (5) has at least one anchoring means along the circumferential direction and/or along its length,
and wherein the anchoring means is formed by a tab (8) that is provided by one or several cuts or recesses that is/are introduced into the strip (5)
wherein the tab (8) has a root (9) via which it is connected to the strip (5) radially movably, and
wherein the root (9) of each of the tabs (8) is
- continually adjacent to a head section (6) or foot section (7) or
- offset against the head section (6) or foot section (7) in longitudinal direction.

2. The marking body (1) according to claim 1,
**characterised in that**
the head section (6) is a head collar (6') and the foot section (7) is a foot collar (7'), and/or wherein
the elongated openings (4) run at least partially in parallel to each other and/or the openings (4) are cuts and/or recesses running axially or helically between the head section (6) and the foot section (7).

3. The marking body (1) according to claim 1 or 2,
**characterised in that**
the shape-memory metal is nitinol and/or
the tubular shape memory structure is a
- sheet (2') bent into a tube (2) that is closed on the long side,
- sheet (2') bent into a tube (2) that still has an opening slit (12) along the long side,
- a tube (2).

4. The marking body (1) according to at least one of the claims 1 to 3,
**characterised in that**
when the least one tab (8) is provided by one or several cuts, the cut or cuts are U-shaped or W-shaped and the at least one tab (8) has a head area (10), wherein the tab (8) has several ends in its head area (10), preferably tips (11) or rounded tips (11').

5. The marking body (1) according to at least one of the claims 1 to 4,
**characterised in that**
the head section (6) and/or the foot section (7) of the tubular structure is/are applied with a closure element, preferably with a cap (13).

6. The marking body (1) according to at least one of the claims 1 to 5,
**characterised in that**
an elongated body is arranged in the tubular structure, preferably a pin or a tube, that has a material selected from the group comprising ferromagnetic metals and alloys, shape-memory metals and polymers, preferably conductive polymers, or combinations of the above.

7. The marking body (1) according to at least one of the claims 1 to 6,
**characterised in that**
the marking body (1) has a housing (100) of a hydrophilic, swelling substance, preferably of a hydrogel or of a collagen, wherein the self-expanding structure (3) of the shape-memory metal is present in the non-application condition in the housing (100).

8. The marking body (1) according to at least one of the claims 1 to 7,
**characterised in that**
in the application condition, the tubular structure has a diameter in the range of 2 mm to 15 mm, preferably in the range of 3 mm to 8 mm, in its most widely protruding point, and a length in the range of 2 mm to 15 mm, preferably in the range of 4 mm to 9 mm.

9. A method for producing a marking body (1) according to at least one of the claims 1 to 8,
**comprising the steps**
a) providing a semi-finished product in the form of a tube (2) or in the form of a sheet (2') of a shape-memory metal,
b) introducing the at least two elongated openings (4) along the longitudinal side of the semi-finished product between the head section (6) and the foot section (7), and, if the semi-finished product is present in the form of a sheet, b') bending of the sheet (2') along its longitudinal axis until it has a tubular structure,
c) using a tool, gripping at the head section (6) and the foot section (7), and applying an axial force while compressing the semi-finished product,
d) heating the semi-finished product to a target temperature and holding the target temperature for a target duration to imprint the shape of the semi-finished product while compressed on the semi-finished product,
e) letting the semi-finished product cool off.

## Revendications

1. Corps de marquage (1) conçu pour le marquage de cellules tumorales dans des tissus, lequel présente une structure auto-expansible (3) à partir d'un métal à mémoire de forme, le métal à mémoire de forme constituant une structure tubulaire qui présente, sur toute sa longueur, au moins deux ouvertures allongées (4) qui ne s'étendent pas plus que d'une partie formant tête (6) vers une partie formant pied (7) de la structure tubulaire,
- la structure tubulaire étant comprimée dans un état d'application et des bandes (5) restantes gonflant à partir du métal à mémoire de forme entre deux ouvertures (4) allongées respectives, adjacentes l'une par rapport à l'autre, et
- la structure tubulaire étant étirée dans un état de non-application, le corps de marquage présentant un moyen d'ancrage,
**caractérisé en ce que**
au moins l'une des bandes (5) présente au moins un moyen d'ancrage le long de la direction circonférentielle et/ou sur toute sa longueur,
et le moyen d'ancrage étant constitué par une lame (8) qui est fournie par une ou plusieurs entailles ou un ou plusieurs évidements, qui est ou sont réalisés dans la bande (5),
la lame (8) présentant une racine (9) par laquelle elle est reliée à la bande (5) en étant déplaçable radialement et la racine (9) de chacune des lames (8)
- étant reliée à la partie formant tête (6) ou la partie formant pied (7) ou
- étant décalée dans le sens longitudinal par rapport à la partie formant tête (6) ou la partie formant pied (7).

2. Corps de marquage (1) conformément à la revendication 1,
**caractérisé en ce que**
la partie formant tête (6) est un collet de tête (6') et la partie formant pied (7) est un collet de pied (7') et/ou
les ouvertures allongées (4) étant au moins en partie parallèles les unes aux autres et/ou les ouvertures (4) entre la partie formant tête (6) et la partie formant pied (7) étant des entailles et/ou des évidements s'étendant en sens axial ou en hélice.

3. Corps de marquage (1) conformément à la revendication 1 ou 2,
**caractérisé en ce que**
le métal à mémoire de forme est du Nitinol et/ou
la structure à mémoire de forme tubulaire est
- une plaque (2') incurvée vers un tube (2), laquelle est fermée en sens longitudinal,
- une plaque (2') incurvée vers un tube (2), laquelle présente encore une fente d'ouverture (12) en sens longitudinal,
- un tube (2).

4. Corps de marquage (1) conformément au moins à l'une des revendications 1 à 3,
**caractérisé en ce que**
lorsqu'au moins une lame est fournie par l'entaille/les entailles, l'entaille ou les entailles sont en forme de U ou en forme de W et au moins une lame (8) présente une zone formant tête (10), la lame présentant plusieurs extrémités, préférablement des pointes (11) ou des pointes arrondies (11'), sur cette zone formant tête (10).

5. Corps de marquage (1) conformément au moins à l'une des revendications 1 à 4,
**caractérisé en ce que**
la partie formant tête (6) et/ou la partie formant pied (7) de la structure tubulaire est dotée ou sont dotées d'un élément de fermeture, préférablement d'un cache (13).

6. Corps de marquage (1) conformément au moins à l'une des revendications 1 à 5,
**caractérisé en ce que**
un corps allongé, préférablement une tige ou un petit tube, est disposé dans la structure tubulaire,
celui-ci présentant un matériau sélectionné dans le groupe comprenant des métaux et des alliages ferromagnétiques, des métaux à mémoire de forme et des polymères, préférablement des polymères conducteurs, ou des combinaisons des éléments susmentionnés.

7. Corps de marquage (1) conformément au moins à l'une des revendications 1 à 6,
**caractérisé en ce que**
le corps de marquage (1) présente une enceinte (100) composée d'une substance hydrophile, gonflable, préférablement d'un hydrogel ou d'un collagène, la structure auto-expansible (3) en métal à mémoire de forme étant dans l'enceinte (100) dans un état de non-application.

8. Corps de marquage (1) conformément au moins à l'une des revendications 1 à 7,
**caractérisé en ce que**
la structure tubulaire présente, dans l'état d'application, à son endroit le plus gonflé, un diamètre entre 2 mm et 15 mm, préférablement entre 3 mm et 8 mm, et une longueur entre 2 mm et 15 mm, préférablement entre 4 mm et 9 mm.

9. Procédé pour la fabrication d'un corps de marquage (1) conformément au moins à l'une des revendications 1 à 8,
**comprenant les étapes suivantes:**
a) fourniture d'un produit semi-fini sous la forme d'un tube (2) ou sous la forme d'une plaque (2') à partir d'un métal à mémoire de forme,
b) réalisation au moins de deux ouvertures allongées (4) le long du côté longitudinal du produit semi-fini entre la partie formant tête (6) et la partie formant pied (7), des bandes (5) étant constituées entre deux ouvertures allongées (4) respectives, adjacentes l'une par rapport à l'autre, et après l'étape b) fabrication d'au moins une lame (8) comme moyen d'ancrage par la réalisation d'entailles ou d'évidements dans au moins l'une des bandes (5) et, si le produit semi-fini est disponible sous la forme d'une plaque, b') flexion de la plaque (2') le long de son axe longitudinal jusqu'à ce qu'elle présente une structure tubulaire,
c) au moyen d'un outil, engagement dans la partie formant tête (6) et la partie formant pied (7) et application d'une force axiale et compression du produit semi-fini, et comme étape c') flexion de la lame (8) reliée à la bande (5) par une racine (9), dans le sens radial pas plus loin que jusqu'à la racine (9),
d) réchauffement du produit semi-fini à une température prescrite et maintien de la température prescrite pendant une durée prescrite, gaufrage de la forme du produit semi-fini pendant la compression,
e) laisser refroidir le produit semi-fini.
